# EUROPEAN PATENT SPECIFICATION

(11) **EP 1 382 336 B1**
(45) Date of publication and mention of the grant of the patent: **08.09.2010**
(21) Application number: 02720584.8
(22) Date of filing: 24.04.2002
(51) Int. Cl.: A61K 31/4439, A61K 31/00, A61K 31/42, A61P 9/10, C07D 263/32, C07D 401/14, C07D 413/12, C07D 413/14, C07D 417/12, C07D 417/14

(54) **USE OF THE ABC EXPRESSION PROMOTOR PIOGLITAZONE FOR THE TREATMENT OF ARTERIOSCLEROSIS OBLITERANS**
VERWENDUNG DES ABC-EXPRESSIONSPROMOTORS PIOGLITAZON ZUR BEHANDLUNG VON ARTERIOSKLEROSE OBLITERANS
UTILISATION DU PROMOTEUR D'EXPRESSION D'ABC PIOGLITAZONE POUR LE TRAITEMENT DE L'ARTERIOSCLEROSE OBLITERANS

(30) Priority: 25.04.2001 JP 2001128222
(43) Date of publication of application: 21.01.2004
(73) Proprietor: Takeda Pharmaceutical Company Limited, Osaka (JP)
(72) Inventor: SUGIYAMA, Yasuo, Kawanishi-shi, Hyogo 666-0111 (JP); FUSE, Hiromitsu, Tsukuba-shi, Ibaraki 305-0821 (JP); HIRAKATA, Masao, Kobe-shi, Hyogo 651-1143 (JP); TOZAWA, Ryuichi, Toyonaka-shi, Osaka 560-0021 (JP)
(74) Representative: Dossmann, Gérard
(86) International application number: PCT/JP2002/004072
(87) International publication number: WO 2002/087580

(56) References cited:
- EP-A- 1 023 907
- EP-A- 1 229 026
- EP-A- 1 304 121
- EP-A1- 0 193 256
- WO-A-00/61127
- WO-A-98/05331
- WO-A-99/59586
- WO-A1-01/03659
- WO-A1-01/17994
- WO-A1-01/34200
- WO-A1-01/34579
- WO-A1-01/38325
- WO-A1-97/31907
- WO-A1-97/37970
- WO-A1-99/04815
- WO-A1-99/32465
- WO-A1-99/58510
- WO-A2-01/66098
- JP-A- 10 072 371
- US-A- 5 968 960
- US-A- 6 159 997
- CHINETTI G ET AL: "PPARALPHA AND PPARGAMMA ACTIVATORS INDUCE CHOLESTEROL REMOVAL FROM HUMAN MACROPHAGE FOAM CELLS THROUGH STIMULATION OF THE ABC-1 PATHWAY" CIRCULATION, AMERICAN HEART ASSOCIATION, DALLAS, TX, US, vol. 102, no. 18, SUPPL, 31 October 2000 (2000-10-31), pages II-311, XP001018171 ISSN: 0009-7322
- VAMECQ J ET AL: "Medical significance of peroxisome proliferator-activated receptors" LANCET THE, LANCET LIMITED. LONDON, GB, vol. 354, no. 9173, 10 July 1999 (1999-07-10), pages 141-148, XP004825909 ISSN: 0140-6736
- WADE D ET AL: "Regulation of the cholesterol efflux gene, ABCA1" LANCET THE, LANCET LIMITED. LONDON, GB, vol. 357, no. 9251, 20 January 2001 (2001-01-20), pages 161-163, XP004799529 ISSN: 0140-6736
- YOKOYAMA S: "Release of cellular cholesterol: molecular mechanism for cholesterol homeostasis in cells and in the body" BIOCHIMICA AND BIOPHYSICA ACTA. MOLECULAR AND CELL BIOLOGY OF LIPIDS, ELSEVIER, AMSTERDAM, NL, vol. 1529, no. 1-3, 15 December 2000 (2000-12-15), pages 231-244, XP004277461 ISSN: 1388-1981
- AKHIL A. PARULKAR ET AL.: "Nonhypoglycemic effects of thiazolidinediones" ANNALS OF INTERNAL MEDICINE, vol. 134, 2 January 2001 (2001-01-02), pages 61-71, XP002439773
- MOLECULAR CELL vol. 7, no. 1, 2001, pages 161 - 171, XP002953879
- NATURE MEDICINE vol. 7, no. 1, 2001, pages 53 - 58, XP002953880
- DIABETES vol. 46, no. 8, 1997, pages 1319 - 1327, XP002950465
- BIOCHEM. BIOPHYS. RES. COMMUN. vol. 238, no. 2, 1997, pages 606 - 611, XP002953881
- PEULER J D ET AL: "DIFFERENTIAL INHIBITORY EFFECTS OF ANTIDIABETIC DRUGS ON ARTERIAL SMOOTH MUSCLE CELL PROLIFERATION" AMERICAN JOURNAL OF HYPERTENSION, NEW YORK, NY, US, vol. 9, 1 February 1996 (1996-02-01), pages 188-192, XP000997538
- 'Arteriosclerosis', [Online] DOCSTOC Retrieved from the Internet: <URL:http://www.docstoc.com/docs/11718516/A rteriosclerosis> [retrieved on 2009-10-16]
- DATABASE EMBASE [Online] ELSEVIER SCIENCE PUBLISHERS, AMSTERDAM, NL 1995 TANI T. ET AL: 'Primary and restenotic lesions obtained from patients with arteriosclerosis obliterans by directional atherectomy: Histopathological and immunocytochemical study' Database accession no. EMB-1995095520 & JAPANESE JOURNAL OF INTERVENTIONAL CARDIOLOGY 1995 JP, vol. 10, no. 1, 1995, pages 26-30, ISSN: 0914-8922
- GORENNE ISABELLE ET AL: "Vascular smooth muscle cell senescence in atherosclerosis" CARDIOVASCULAR RESEARCH, vol. 72, no. 1, October 2006 (2006-10), pages 9-17, ISSN: 0008-6363
- INTERNET ARTICLE, [Online] Retrieved from the Internet: <URL:http://www.answers.com/topic/arteriosc lerosis-obliterans> [retrieved on 2008-08-11]
- M.H. BEERS, R. BERKOW: "The Merck Manual of Diagnosis and Therapy, 17th Edition" 1999, MERCK RESEARCH LABORATORIES , WHITEHOUSE STATION N.J. * page 1654 - page 1658 *

### SEQUENCE LISTING

<110> Takeda Chemical Industries, Ltd.
<120> ABC Expression Promoting Agent
<130> 2907WO0P
<150> JP 2001-128222
<151> 2001-04-25
<160> 24
<210> 1
<211> 23
<212> DNA
<213> Artificial Sequence
<220>
<223> Sense Strand Primer
<400> 1
   gctgagctac ccaccctatg aac□@□@23
<210> 2
<211> 21
<212> DNA
<213> Artificial Sequence
<220>
<223> Anti Sense Strand Primer
<400> 2
   taatcccctg aacccaagga a 21
<210> 3
<211> 27
<212> DNA
<213> Artificial Sequence
<220>
<223> Taq Man probe
<400> 3
   tgccattttc caaataaagc catgccc 27
<210> 4
<211> 21
<212> DNA
<213> Artificial Sequence
<220>
<223> Sense Strand primer
<400> 4
   acacctacat gcgtcgcaag t 21
<210> 5
<211> 21
<212> DNA
<213> Artificial Sequence
<220>
<223> Antisense Strand primer
<400> 5
   tcttgccgct tcagtttctt c 21
<210> 6
<211> 28
<212> DNA
<213> Artificial Sequence
<220>
<223> Taq Man probe
<400> 6
   tgtgtcctgt cagaagaaca gatccgcc 28
<210> 7
<211> 22
<212> DNA
<213> Artificial Sequence
<220>
<223> Sense Strand primer
<400> 7
   tggaaaatgc caagctgtac ct 22
<210> 8
<211> 20
<212> DNA
<213> Artificial Sequence
<220>
<223> Anti Sense Strand Primer
<400> 8
   cggattttgt acctgaggac 20
<210> 9
<211> 26
<212> DNA
<213> Artificial Sequence
<220>
<223> Taq Man probe
<400> 9
   ttcatctccc tccgcctcat tgccta 26
<210> 10
<211> 21
<212> DNA
<213> Artificial Sequence
<220>
<223> Sense Strand primer
<400> 10
   agcaacagtg taacaggcgc t□@□@21
<210> 11
<211> 21
<212> DNA
<213> Artificial Sequence
<220>
<223> Antisense Strand primer
<400> 11
   acgatggcca gctcagtaaa g□@□@21
<210> 12
<211> 20
<212> DNA
<213> Artificial Sequence
<220>
<223> Taq Man probe
<400> 12
   ctcagaccgc ctgcgcgtca□@□@20
<210> 13
<211> 19
<212> DNA
<213> Artificial Sequence
<220>
<223> sense Strand primer
<400> 13
   tcaggaggcc atgatggtg□@□@19
<210> 14
<211> 19
<212> DNA
<213> Artificial Sequence
<220>
<223> Antisense Strand Primer
<400> 14
   cccgtctgcc ttcatcctt□@□@19
<210> 15
<211> 21
<212> DNA
<213> Artificial Sequence
<220>
<223> Taq Man probe
<400> 15
   ccgcgcatct gaagctgcag g□@□@21
<210> 16
<211> 22
<212> DNA
<213> Artificial Sequence
<220>
<223> Sense Strand primer
<400> 16
   agacctcctt cttccaggct tt□@□@22
<210> 17
<211> 23
<212> DNA
<213> Artificial Sequence
<220>
<223> Antisense Strand primer
<400> 17
   gctgcacatc actcagaatt tca□@□@23
<210> 18
<211> 26
<212> DNA
<213> Artificial Sequence
<220>
<223> Taq Man probe
<400> 18
   tcaccacgaa aatctccaga ggcacc□@□@26
<210> 19
<211> 21
<212> DNA
<213> Artificial Sequence
<220>
<223> Sense Strand Primer
<400> 19
   gcagctgctc tggttctcat g□@□@21
<210> 20
<211> 23
<212> DNA
<213> Artificial Sequence
<220>
<223> Antisense Strand Primer
<400> 20
   ttcttgccag tggtgtaaca ttg□@□@23
<210> 21
<211> 21
<212> DNA
<213> Artificial Sequence
<220>
<223> Taq Man probe
<400> 21
   cggcagatgc agcgaagagg c□@□@21
<210> 22
<211> 18
<212> DNA
<213> Artificial Sequence
<220>
<223> Sense Strand primer
<400> 22
   agcccttggc tgagcaaa□@□@18
<210> 23
<211> 19
<212> DNA
<213> Artificial Sequence
<220>
<223> Antisense Strand primer
<400> 23
   gtcgcaggca gtgaaccat□@□@19
<210> 24
<211> 26
<212> DNA
<213> Artificial sequence
<220>
<223> Taq Man probe
<400> 24
   tgctatcact gctgggtgca aaacca□@□@26

## Description

The present invention relates to an ABCA1 mRNA expression promoting agent, an LXRα mRNA expression-promoting agent, an ABCG1 mRNA expression-promoting agent, a cholesterol efflux-promoting agent, a cholesteryl ester accumulation-inhibiting agent, an ACAT (acyl-coenzyme A: cholesterol acyl transferase)-1 mRNA expression-inhibiting agent and a CEH (cholesteryl ester hydrolase) mRNA expression-promoting agent that are useful for control of cholesterol distribution in the body. In particular, it relates to pioglitazone or a salt thereof for use in the treatment of arteriosclerosis obliterans, as defined in the claims.

AHCA1 is a member of the ATP-binding cassette-transporter family and is involved in control of apoA1-mediated cholesterol efflux from macrophages. ABCG1 is also involved in control of apoA1-mediate cholesterol efflux.

LXR-α is a nuclear receptor that is activated by oxysterol and mediates the transcription induction of ABCA1 and ABCG1 gene promoters.

Nature Medicine, Vol 7, No. 1, pp. 53-58 discloses that 1) PPAR-α and PPAR-γ activators induce expression of a gene encoding ABCA1, 2) PPAR-α and PPAR-γ activators increase cholesterol efflux from normal macrophages induced by apoAl, and 3) PPAR-α and PPAR-γ activators induce LXR-α gene expression in human macrophages, and exemplifies rosiglitazone and troglitazone as PPAR-γ activators.

Parulkar A.A. discloses nonhypoglycemic effects of thiazolidinediones (Parulkar A.A. Pendergrass M.L., Granda-Ayala R. Lee T.R. Fonseca V.A. nonhypoglycemic effects of thiazolidinediones. Ann Intern Med 2001; 13461-71). This document teaches that thiazolidinediones activate PPAR-γ, promoting uptake of oxidized LDL cholesterol by macrophages and that troglitazone inhibits expression and functional activity of matrix metalloproteinase-9 in human monocytes.

In Peuler J.D., Phare M.S., Iaanucci A.R., Hodorek, M.J. Differential inhibitory Effects of antidiabetic drugs on arterial smooth muscle cell proliferation. Am. J. Hypertens. 1996; 9:188-192, the inhibitory effects of pioglitazone on smooth muscle cells derived from arterioles of healthy and young rats grown for 14 days in foetal calf serum are disclosed.

There is need for development of an ABCA1 mRNA expression-promoting agent, an LXRα mRNA expression-promoting agent, an ABCG1 mRNA expression-promoting agent, a cholesterol efflux-promoting agent, a cholesteryl ester accumulation - inhibiting agent, an ACAT-1 mRNA expression - inhibiting agent and a CEH mRNA expression - promoting agent which have excellent ability to control cholesterol distribution in the body and have low toxicity.

The present invention relates to pioglitazone or a salt thereof, an agent comprising pioglitazone or a salt thereof for use in treatment of arteriosclerosis obliterans,
to the use of pioglitazone or a salt thereof for the manufacture of a medicament for treating arteriosclerosis obliterans,

The agent is :
- an ABCA1 mRNA expression-promoting agent comprising pioglitazone or a salt thereof;
- an LXRα mRNA expression-promoting agent comprising pioglitazone or a salt thereof;
- an ABCG1 mRNA expression-promoting agent comprising pioglitazone or a salt thereof;
- a cholesterol efflux-promoting agent comprising pioglitazone or a salt thereof;
- a cholesteryl ester accumulation-inhibiting agent comprising pioglitazone or a salt thereof;
- an ACAT-1 mRNA expression-inhibiting agent comprising PPARγ modulator , wherein the PPARγ modulator is pioglitazone or a salt thereof;
- a CEH mRNA expression-promoting agent comprising a PPARγ modulator, wherein the PPARγ modulator is pioglitazone or a salt thereof;

Salts of pioglitazone used in the present invention include pharmacologically acceptable salts such as salts with inorganic bases, salts with organic bases, salts with inorganic acids, salts with organic acids, salts with basic or acidic amino acids.

Preferable examples of salts with inorganic bases include salts with alkaline metals such as sodium and potassium, alkali earth metals such as calcium and magnesium, aluminum and ammonium.

Preferable examples of salts with organic bases include salts with trimethylamine, triethylamine, pyridine, picoline, ethanolamine, diethanolamine, triethanolamine, dicyclohexylamine and N,N-dibenzylethylenediamine.

Preferable examples of salts with inorganic acids include salts with hydrochloric acid, hydrobromic acid, nitric acid, sulfuric acid and phosphoric acid.

Preferable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Preferable examples of salts with basic amino acids include salts with arginine, lysine and ornithine. Preferable examples of salts with acidic amino acids include salts with aspartic acid and glutamic acid.

Among the above-mentioned salts, the salt with inorganic acid is preferable and hydrochloride is especially preferable.

In the present invention, although an ABCA1 mRNA expression-promoting agent, an LXRα mRNA expression-promoting agent, an ABCG1 mRNA expression-promoting agent, a cholesterol efflux-promoting agent and a cholesteryl ester accumulation-inhibiting agent, each of which comprises "pioglitazone or a salt thereof", may be the active ingredient "pioglitazone or a salt thereof" itself, they are generally produced by mixing the active ingredient and a pharmacologically acceptable carrier according to a method known per se [e.g. a conventional method in the art of pharmaceutical technique, for example, a method disclosed in The Pharmacopoeia of Japan (e.g., 13th edition) ].

Here, the dosage forms of the ABCA1 mRNA expression-promoting agent, LXRα mRNA expression-promoting agent, ABCG1 mRNA expression-promoting agent, cholesterol efflux-promoting agent and cholesteryl ester accumulation inhibiting agent include an oral preparation such as tablets (including sublingual tablets and buccal disintegrating tablets), capsules (including soft capsules and microcapsules), powders, granules, troches and syrups; and a parenteral preparation such as injections (e.g., subcutaneous injections, intravenous injections, intramuscular injections, intraperitoneal injections or drops), an external preparation (e.g., a percutaneous preparation or ointments), suppositories (e.g., rectal suppositories or vaginal suppositories), pellets, a nasal agent, a transpulmonary agent (inhalations) or eye drops. These preparations may be a controlled release preparation such as a quick release preparation or a sustained release preparation (e.g., sustained release microcapsules).

Hereinafter production methods for an oral preparation and a parenteral preparation are specifically explained.

The oral preparation is produced by adding, for example, an excipient (e.g., lactose, sucrose, starch, D-mannitol, xylitol, sorbitol, erithritol, crystalline cellulose or light anhydrous silicic acid), a disintegrator (e.g., calcium carbonate, starch, carboxymethyl cellulose, carboxymethyl cellulose calcium, low substituted hydroxypropyl cellulose, croscarmellose sodium, carboxymethyl starch sodium or light anhydrous silicic acid), a binder (e.g., alpha starch, gum arabic, carboxymethyl cellulose, hydroxypropyl cellulose, hydroxypropylmethyl cellulose, polyvinylpyrrolidone, crystalline cellulose, methylcellulose, sucrose, D-mannitol, trehalose or dextrin) or a lubricant (e.g., talc, magnesium stearate, calcium stearate, colloidal silica or polyethyleneglycol 6000) to an active ingredient and compression molding the mixture. In order to accelerate dissolution of the active ingredient, acids such as hydrochloric acid, phosphoric acid, malonic acid, succinic acid, DL-malic acid, tartaric acid, maleic acid, fumaric acid or citric acid or bases such as sodium carbonate, sodium hydrogencarbonate, sodium citrate or sodium tartrate may be also added to the oral preparation.

Furthermore, for the purpose of masking of taste, enteric property or sustained-release, the oral preparation may be coated according to a method known per se. The coating agent used includes an enteric polymer (e.g., acetate phthalate cellulose, methacrylic acid copolymer L, methacrylic acid copolymer LD, methacrylic acid copolymer S, hydroxypropylmethyl cellulose phthalate, hydroxypropylmethyl cellulose acetate succinate or carboxymethylethyl cellulose), a gastric-soluble polymer (e.g., polyvinyl acetal diethylaminoacetate or aminoalkyl methacrylate copolymer E), a watersoluble polymer (e.g., hydroxypropyl cellulose or hydroxypropylmethyl cellulose), a water-insoluble polymer (e.g., ethyl cellulose, aminoalkyl methacrylate copolymer RS or ethyl acrylate-methyl methacrylate copolymer) or a wax. During the coating, a plasticizer such as polyethyleneglycol, an opacifying agent such as titanium oxide or iron sesquioxide may be used together with the coating agent.

The injection is produced by dissolving, suspending or emulsifying the active ingredient in an aqueous solvent (e.g., distilled water, saline or Ringer's solution) or an oily solvent (e.g., a vegetable oil such as olive oil, sesame oil, cottonseed oil or corn oil ; propylene glycol, macrogol or tricaprylin) together with a dispersing agent (e.g., Tween 80 (manufactured by Atras Powder, USA), HCO 60 (manufactured by Nikko Chemicals Co., Ltd.), polyethyleneglycol, carboxymethylcellulose or sodium alginate), a preserving agent (e.g., methylparaben, propylparaben, benzyl alcohol, chlorobutanol or phenol), an isotonicity agent (e.g., sodium chloride, glycerine, D-sorbitol, D-mannitol, xylitol, glucose or fructose).

During the above-mentioned process, if desired, additives such as a dissolving agent (e.g., sodium salicylate, sodium acetate, polyethyleneglycol, propyleneglycol, D-mannitol, trehalose, benzyl benzoate, ethanol, trisaminomethane, cholesterol, triethanolamine, sodium carbonate or sodium citrate), a suspending agent (e.g., a surfactant such as stearyl triethanolamine, sodium laurylsulfate, laurylaminopropionate, lecithin, benzalkonium chloride, benzethonium chloride and glycerine monostearate; a hydrophilic polymer such as polyvinylalcohol, polyvinylpyrrolidone, carboxymethylcellulose sodium, methylcellulose, hydroxymethylcellulose, hydroxyethylcellulose and hydroxypropylcellulose), a buffer (e.g., a buffer solution such as phosphate, acetate, carbonate and citrate), a stabilizing agent (e.g., human serum albumin), a soothing agent (e.g., propyleneglycol, lidocaine hydrochloride or benzylalcohol) and an antiseptic (e.g., parahydroxybenzoic acid esters, chlorobutanol, benzalkonium chloride, benzylalcohol, phenethylalcohol, dehydroacetic acid or sorbic acid) may be used.

The external preparation is produced by formulating the active ingredient into solid, semi-solid or liquid composition. For example, the solid composition is produced by pulverizing the active ingredient as it is, or by adding an excipient (e.g., lactose, D-mannitol, starch, crystalline cellulose or sucrose), a thickener (e.g., natural rubbers, cellulose derivatives or acrylic acid polymers) to the active ingredient, mixing them and then pulverizing the mixture. The liquid composition is produced almost similarly to the injection. The semi-solid composition is preferably an aqueous or oily gel, or an ointment form. All of these compositions may also contain a pH modulating agent (e.g., phosphoric acid, citric acid, hydrochloric acid or sodium hydroxide), an antiseptic (e.g., parahydroxybenzoic acid esters, chlorobutanol, benzalkonium chloride, benzylalcohol, phenethylalcohol, dehydroacetic acid or sorbic acid).

The suppository is produced by formulating the active ingredient into an oily or aqueous solid, semi-solid or liquid composition. Oily bases used in production of the composition include glyceride of higher fatty acid [e.g., cacao butter, Witepsols (manufactured by Hüls Aktiengesellschaft, Germany)], medium fatty acid triglyceride [e.g., Miglyols (manufactured by Hüls Aktiengesellschaft, Germany)], vegetable oil (e.g., sesame oil, soybean oil or or cottonseed oil). Aqueous bases used in production of the composition include polyethyleneglycols or propyleneglycol. Aqueous gel bases used in production of the composition include natural rubbers, cellulose derivatives, vinyl polymers, or acrylic acid polymers.

In the ABCA1 mRNA expression-promoting agent, LXRα mRNA expression-promoting agent, ABCG1 mRNA expression-promoting agent, cholesterol efflux-promoting agent and cholesteryl ester accumulation-inhibiting agent, the content of the active ingredient "pioglitazone or a salt thereof" is, for example, 0.1 to 100% by weight, preferably 5 to 80% by weight.

The ABCA1 mRNA expression-promoting agent, LXRα mRNA expression-promoting agent, ABCG1 mRNA expression-promoting agent, cholesterol efflux-promoting agent and cholesteryl ester accumulation-inhibiting agent of the present invention have low toxicity, and can be used safely for mammals (e.g., human, mouse, rat, rabbit, dog, cat, cattle, horse, pig, monkey and the like) orally or parenterally.

The dose of the ABCA1 mRNA expression-promoting agent, LXRα mRNA expression-promoting agent, ABCG1 mRNA expression-promoting agent, cholesterol efflux-promoting agent and cholesteryl ester accumulation-inhibiting agent of the present invention may follow the dose of the active ingredient "pioglitazone or a salt thereof", and can be suitably selected depending on the subject to be administered, age and body weight of the subject, condition, administration period, dosage form, administration method and the like.

For example, when the ABCA1 mRNA expression-promoting agent, LXRα mRNA expression-promoting agent, ABCG1 mRNA expression-promoting agent, cholesterol efflux-promoting agent or cholesteryl ester accumulation-inhibiting agent is administered to an adult (body weight 50 kg), the daily dose is generally 0.01 to 1000 mg, preferably 0.1 to 600 mg of the active ingredient "pioglitazone or a salt thereof". Such daily dose may be administered in one to several doses.

Specifically, when the ABCA1 mRNA expression-promoting agent, LXRα mRNA expression-promoting agent, ABCG1 mRNA expression-promoting agent, cholesterol efflux-promoting agent or cholesteryl ester accumulation-inhibiting agent comprising the "pioglitazone or a salt thereof (preferably hydrochloride)" is orally administered to an adult (body weight 50 kg), the daily dose is generally 7.5 to 60 mg, preferably 15 to 45 mg of the "pioglitazone or a salt thereof". Such daily dose may be administered in one or two doses.

The ABCA1 mRNA expression-promoting agent of the present invention can increase the intracellular content of ABCA1. Thus increased ABCA1 can bind to apoprotein (e.g., apoAI, apoAII and the like) or apolipoprotein (e.g., high density lipoprotein, HDL) present in a living body to carry intracellular cholesterol out of cells. The cholesterol thus carried out of cells is then transported to a tissue having low cholesterol content. Therefore, the ABCA1 mRNA expression-promoting agent of the present invention is useful for control of cholesterol distribution in the body.

Therefore, based on the intracellular cholesterol-efflux effect, the ABCA1 mRNA expression-promoting agent of the present invention is useful as, an agent for treatment of arteriosclerosis obliterans;.

The LXRα mRNA expression-promoting agent of the present invention can increase the intracellular content of LXRα. Since the LXRα allows ABCA1 mRNA to be expressed, the LXRα mRNA expression-promoting agent of the present invention is useful as an agent for treatment of arteriosclerosis obliterans as disease for which the ABCA1 mRNA expression-promoting agent of the present invention is useful.

The ABCG1 mRNA expression-promoting agent of the present invention can increase the intracellular content of ABCG1. Thus-increased ABCG1 can bind to apoprotein (e.g., apoAI, apoAII and the like) or apolipoprotein (e.g., high density lipoprotein, HDL) present in a living body to carry intracellular cholesterol out of cells. The cholesterol thus carried out of cells is then transported to a tissue having low cholesterol content. Therefore, the ABCG1 mRNA expression-promoting agent of the present invention is useful as an agent for treatment of arteriosclerosis obliterans as disease for which the ABCA1 mRNA expression-promoting agent of the present invention is useful.

The cholesterol efflux-promoting agent and cholesteryl ester accumulation-inhibiting agent of the present invention are useful as an agent treatment of arteriosclerosis obliterans as disease for which the ABCA1 mRNA expression-promoting agent of the present invention is useful.

The "PPARγ modulator" is "pioglitazone or a salt thereof (preferably hydrochloride)".

The dosage form of the ABCG1 mRNA expression-promoting agent includes dosage forms similar to those of the ABCA1 mRNA expression promoting agent as mentioned above.

In the ABCG1 mRNA expression-promoting agent, the content of the active ingredient is, for example, 0.1 to 100% by weight, preferably 5 to 80% by weight.

The ABCG1 mRNA expression-promoting agent has low toxicity, and can be safely used for mammals (e.g., human, mouse, rat, rabbit, dog, cat, cattle, horse, pig, monkey and the like) orally or parenterally.

The dose of the ABCG1 mRNA expression-promoting agent may follow the dose of the active ingredient, and can be suitably selected depending on the subject to be administered, age and body weight of the subject, condition, administration period, dosage form, administration method and the like.

For example, the ABCG1 mRNA expression promoting agent is orally administered to an adult (body weight 50 kg), the daily dose is generally 0.01 to 1000 mg, preferably 0.1 to 600 mg of the active ingredient. Such daily dose may be administered in one to several doses.

The ABCG1 mRNA expression-promoting agent can increase the intracellular content of ABCG1. Thus increased ABCG1 can bind to apoprotein (e.g., apoAI, apoAII and the like) or apolipoprotein (e.g., high density lipoprotein, HDL) present in a living body to carry intracellular cholesterol out of cells. The cholesterol thus carried out of cells is then transported to a tissue having low cholesterol content. Therefore, the ABCG1 mRNA expression-promoting agent of the present invention is useful as an agent for treatment of arteriosclerosis obliterans as disease for which the ABCA1 mRNA expression-promoting agent of the present invention is useful.

Although the ACAT-1 mRNA expression-inhibiting agent and CEH mRNA expression-promoting agent of the present invention may be the active ingredient "PPARγ modulator" itself, they are generally produced by mixing the active ingredient and a pharmacologically acceptable carrier according to a method known per se [e.g., a conventional method in the art of pharmaceutical technique, for example, a method disclosed in The Pharmacopoeia of Japan (e.g., 13th edition) and the like].

The dosage form of the ACAT-1 mRNA expression-inhibiting agent and CEH mRNA expression-promoting agent includes dosage forms similar to those of the ABCA1 mRNA expression promoting agent and the like as mentioned above.

In the ACAT-1 mRNA expression-inhibiting agent and CEH mRNA expression-promoting agent, the content of the active ingredient "PPARγ modulator" is, for example, 0.1 to 100% by weight, preferably 5 to 80% by weight.

The ACAT-1 mRNA expression-inhibiting agent and CEH mRNA expression-promoting agent have low toxicity, and can be safely used for mammals (e.g., human, mouse, rat, rabbit, dog, cat, cattle, horse, pig, monkey and the like) orally or parenterally.

The dose of the ACAT-1 mRNA expression-inhibiting agent and CEH mRNA expression-promoting agent may follow the dose of the active ingredient "PPARγ modulator", and can be suitably selected depending on the subject to be administered, age and body weight of the subject, condition, administration period, dosage form, administration method and the like.

For example, the ACAT-1 mRNA expression-inhibiting agent or CEH mRNA expression-promoting agent is administered to an adult (body weight 50 kg), the daily dose is generally 0.01 to 1000 mg, preferably 0.1 to 600 mg of the active ingredient "PPARγ modulator". Such daily dose may be administered in one to several doses.

Specifically, the ACAT-1 mRNA expression-inhibiting agent or CEH mRNA expression-promoting agent comprising "pioglitazone or a salt thereof (preferably hydrochloride)" as the "PPARγ modulator" is orally administered to an adult (body weight 50 kg), the daily dose is generally 7.5 to 60 mg, preferably 15 to 45 mg of the "pioglitazone or a salt thereof". Such daily dose may be administered in one or two doses.

The ACAT-1 mRNA expression-inhibiting agent and CEH mRNA expression-promoting agent of the present invention are useful as an agent for treatment of arteriosclerosis obliterans as disease for which the ABCA1 mRNA expression-promoting agent is useful.

The ABCA1 mRNA expression-promoting agent, LXRα mRNA expression-promoting agent, ABCG1 mRNA expression-promoting agent, cholesterol efflux-promoting agent, cholesteryl ester accumulation-inhibiting agent, ACAT-1 mRNA expression-inhibiting agent and CEH mRNA expression-promoting agent of the present invention (hereinafter sometimes abbreviated as the agents of the present invention) can be used in combination with concomitant agents that do not adversely affect the agents of the present invention, for the purpose of "enhancing the effect of the agents of the present invention", "decreasing an amount of the agents of the present invention to be used" or "decreasing side effect of the agents of the present invention". The concomitant agents may be a low molecular compound or a high molecular protein, polypeptide, antibodies or vaccines.

Such concomitant agents include an "agent for treatment of diabetes mellitus", "agent for treatment of diabetic complication", "antiobestic agent", "agent for treatment of hypertension", "agent for treatment of hyperlipidemia", "diuretic", "antithrombotic agent" or "agent for treatment of Alzheimer disease". Two or more of these concomitant agents may be used in combination.

The above mentioned "agent for treatment of diabetes mellitus" includes an insulin secretion promoting drug, biguanide, insulin, α-glucosidase inhibitor and β3 agonist.

The insulin secretion-promoting drug includes sulfonylurea. Specific examples of sulfonylurea include tolbutamide, chlorpropamide, tolazamide, acetohexamide, glyclopyramide and an ammonium salt thereof, glibenclamide, gliclazide, 1-butyl-3-methanilylurea, carbutamide, glibornuride, glipizide, gliquidone, glisoxepide, glybuthiazole, glybuzole, glyhexamide, glymidine, glypinamide, phenbutamide, tolcyclamide, and glimepiride.

Besides the above-mentioned drugs, the insulin secretion-promoting drug includes N-[[4-(1-methylethyl)cyclohexyl]carbonyl]-D-phenylalanine [Nateglinide], (2S)-2-benzyl-3-(cis-hexahydro-2-isoindolinylcarbonyl)propionic acid calcium dihydrate [Mitiglinide], Repaglinide, GLP (Glucagon-like peptide)-1, GLP-1(7-36)-amide, V8-GLP-1 (LY-307161), Exendin-4 (AC-2993), DPP-728-A, V-411, and JT-608.

The biguanide includes phenformin, metformin and buformin.

The insulin includes animal insulin extracted from pancreas of cattle or pig; semi-synthetic human insulin enzymatically synthesized from insulin extracted from pancreas of pig; and human insulin genetic technologically synthesized using Escherichia coli or yeast. As the insulin, insulin zinc containing 0.45 to 0.9(w/w)% of zinc; protamine insulin zinc produced from zinc chloride, sulfuric acid protamine and insulin may be used. Furthermore, the insulin may be a fragment or derivative thereof (e.g., INS-1).

The insulin includes various types such as super immediate-acting type, immediate-acting type, biphasic type, medium type and long-acting type, and can be selected depending on the pathology of a patient.

The α-glucosidase inhibitor includes acarbose, voglibose, miglitol and emiglitate.

The β3 agonist (β3 adrenaline receptor agonist) includes SR-58611-A, SB-226552 and AZ40140.

Besides the above-mentioned drugs, the "agent for treatment of diabetes mellitus" includes ergoset, pramlintide, leptin, BAY-27-9955 and T-1095.

The "agent for treatment of diabetic complication" includes an aldose reductase inhibitor, glycation inhibitor and protein kinase C inhibitor.

The aldose reductase inhibitor includes torlestat; eparlestat; 3,9-dihydro-2,8-diisopropyl-3-thioxo-2H-1,9-benzoxazine-4-acetic acid; imirestat; zenarestat; 6-fluoro-2,3-dihydro-2',5'-dioxo-spiro[4H-1-benzopyran-4,4'-imidazolidine]-2-carboxamide (SNK-860); zopolrestat; sorbinil; 1-[(3-bromo-2-benzofuranyl)sulfonyl]-2,4-imidazolidinedione (M-16209) : CT-112; NZ-314; and ARI-509.

The glycation inhibitor includes pimagedine.

The protein kinase C inhibitor includes NGF and LY-333531.

Besides the above-mentioned drugs, the "agent for treatment of diabetic complication" includes alprostadil, tiapride hydrochloride, cilostazol, mexiletine hydrochloride, ethyl icosapentate, memantine, pimagedline (ALT-711), neurotrophic factor (e.g., nerve growth factor (NGF), brain-derived nerve growth factor (BDNF), neurotrophic factor 3 (NT-3)), an increasing agent or enhancing agent thereof).

The "antiobestic agent" includes a lipase inhibitor and anorexic.

The lipase inhibitor includes orlistat.

The anorexic includes dexfenfluramine, fluoxetine, sibutramine, and biamine.

The "agent for treatment of hypertension" includes an angiotensin-converting enzyme inhibitor, calcium antagonist, potassium channel opener, and angiotensin II antagonist.

The angiotensin-converting enzyme inhibitor includes captopril, enalapril, alacepril, delapril, ramipril, lisinopril, imidapril, benazepril, ceronapril, silazapril, enalaprilate, fosinopril, movertipril, perindopril, quinapril, spirapril, temocapril, trandolapril, and manidipine.

The calcium antagonist includes nifedipine, amlodipine, efonidipine, and nicardipine.

The potassium channel opener includes levcromakalim, L-27152, AL-0671, and NIP-121.

The angiotensin II antagonist includes losartan, candesartan cilexetil, varsartan, irbesartan, (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate (CS-866), and E4177.

The "agent for treatment of hyperlipidemia" includes an HMG-CoA reductase inhibitor, fibrate compound, squalene synthase inhibitor, and ACAT inhibitor.

The HMG-CoA reductase inhibitor includes pravastatin, simvastatin, lovastatin, atorvastatin, fluvastatin, lipantil, cerivastatin, itavastatin, ZD-4522 or a salt thereof (e.g., sodium salt).

The fibrate compound includes bezafibrate, beclobrate, binifibrate, ciprofibrate, clinofibrate, clofibrate, clofibric acid, etofibrate, fenofibrate, gemfibrozil, nicofibrate, pirifibrate, ronifibrate, simfibrate, and theofibrate.

The squalene synthase inhibitor includes a compound described in WO97/10224 such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid.

The ACAT inhibitor includes avasimibe, and eflucimibe.

Beside the above-mentioned drugs, the "agent for treatment of hyperlipidemia" includes anion exchange resin (e.g., cholestyramine), probucol, nicotinic acid agent (e.g., nicomol, niceritrol), ethyl icosapentate, vegetable sterol (e.g., soysterol, γ-oryzanol).

The "diuretic" includes a xanthine derivative preparation, thiazide preparation, antialdosterone preparation, carbonic anhydrase inhibitor and chlorobenzenesulfonamide preparation.

The xanthine derivative preparation includes theobromine and sodium salicylate, theobromine and calcium salicylate.

The thiazide preparation includes ethiazide, cyclopenthiazide, trichloromethiazide, hydrochlorothiazide, hydroflumethiazide, benzylhydrochlorothiazide, penflutizide, polythiazide, and methyclothiazide.

The antialdosterone preparation includes spironolactone, and triamterene.

The carbonic anhydrase inhibitor includes acetazolamide.

The chlorobenzenesulfonamide preparation includes chlorotalidone, mefruside, and indapamide.

Besides the above-mentioned drugs, the "diuretic" includes azosemide, isosorbide, ethacrynic acid, piretanide, bumetanide, and furosemide.

The "antithrombotic agent" includes heparin, warfarin, antithrombin agent, thrombolytic agent, and platelet aggregation suppressing agent (anti-platelet agent).

The heparin includes heparin sodium, heparin calcium, and dalteparin sodium.

The warfarin includes warfarin potassium.

The antithrombin agent includes aragatroban.

The thrombolytic agent includes urokinase, tisokinase, alteplase, nateplase, monteplase, and pamiteplase.

The platelet aggregation-suppressing agent includes ticlopidine hydrochloride, cilostazol, ethyl icosapentate, beraprost sodium, and sarpogrelate hydrochloride.

The "agent for treatment of Alzheimer disease" includes tacrine, donepezil, rivastigmine, and galantamine.

The stages for administration of the agents of the present invention and the concomitant agents are not specifically limited, and both agents may be simultaneously administrated to the subject to be administered, or may be individually administrated at an interval. The dose of the concomitant agents may follow the clinically used dose, and can be suitably selected depending on the subject to be administered, age and body weight of the subject, condition, administration period, dosage form, administration method, and combination.

The dosage form of the concomitant agents is not specifically limited as long as the agents of the present invention and the concomitant agents are in combination when they are administered. Such dosage form include 1) administration of a single preparation obtained by formulating the agent of the present invention and the concomitant agent together, 2) simultaneous administration of two kinds of preparations via the same route, wherein the two kinds of preparations are obtained by separately formulating the agent of the present invention and the concomitant agent, 3) administration of two kinds of preparations at an interval via the same route, wherein the two kinds of preparations are obtained by separately formulating the agent of the present invention and the concomitant agent, 4) simultaneous administration of two kinds of preparations via the different routes, wherein the two kinds of preparations are obtained by separately formulating the agent of the present invention and the concomitant agent, 5) administration of two kinds of preparations at an interval via the different routes, wherein the two kinds of preparations are obtained by separately formulating the agent of the present invention and the concomitant agent, (e.g., administration in the order of the agent of the present invention and then the concomitant agent, or in the reverse order).

The combination ratio of the agent of present invention and the concomitant agent can be suitably selected depending on the subject to be administered, age and body weight of the subject, condition, administration period, dosage form and administration method.

For example, the concomitant agent may be used in an amount of 0.0001 to 10000 part(s) by weight per 1 part by weight of the agent of the present invention.

The activity of the ABCA1 mRNA expression-promoting agent of the present invention can be confirmed, for example, according to the following method.

Human-derived cells are suspended in a PMA (phorbol 12-myristate 13-acetate)-containing RPMI1640 medium (containing serum), dispensed into plates and cultured until differentiation.

The medium is removed, and a β-VLDL-OA-BSA-containing RPMI1640 medium to which the test compound has been added, is added to the plate and cultured.

Whole RNA is extracted from the cultured cells using RNeasy Mini Kit (QIAGEN). Then, cDNA is synthesized by reverse-transcription reaction of the RNA using TaqMan Reverse Transcription Reagents (Applied Biosystems). Using the obtained reverse-transcription reaction liquid as template DNA and according to TaqMan PCR method using TaqMan Universal PCR Master Mix, expression amounts of ABCA1 mRNA and glyceraldehyde-3-phosphate dehydrogenase (G3PDH) mRNA as an internal standard are measured.

In the above mentioned method, for the measurement of the expression amount of ABCA1 mRNA, oligo DNAs (designed using the Primer Express Program provided by Applied Biosystems) represented by
5'-GCTGAGCTACCCACCCTATGAAC-3' (SEQ ID NO: 1),
5'-TAATCCCCTGAACCCAAGGAA-3' (SEQ ID NO: 2) and
5'-TGCCATTTTCCAAATAAAGCCATGCCC-3' (SEQ ID NO: 3), are used as a sense strand primer, an antisense strand primer and a TaqMan probe, respectively.

For the TaqMan probe used for the measurement of the expression amount of ABCA1 mRNA, 6-carboxyfluorescein (FAM) is used as a reporter dye, and 6-carboxy-N,N,N',N'-tetramethylrhodamine (TAMRA) is used as a quencher dye.
For that purpose, for example, 6FAM is added to the 5'-terminus, and TAMRA is added to the 3'-terminus.

The measurement of the G3PDH mRNA expression is carried out using TaqMan G3PDH Control Reagents (Applied Biosystems), and VIC is used as a reporter dye and TAMRA is used as a quencher dye.

Real-time quantitative PCR reaction is carried out by 40 cycles, wherein one cycle comprises reaction of 50°C for 2 minutes and at 95°C for 10 minutes, followed by keeping at 95°C for 15 seconds and reaction at 60°C for 1 minute, using ABI PRISM 7700 Sequence Detector (Applied Biosystems).

Based on the ratio of the expression amount of ABCA1 mRNA relative to the expression amount of G3PDH mRNA, ABCA1 mRNA expression promoting activity of test compounds can be confirmed.

The activity of the LXRα mRNA expression-promoting agent of the present invention can be confirmed, for example, according to the following method.

The activity of the LXRα mRNA expression-promoting agent can be confirmed by a method similar to that for the above-mentioned ABCA1 mRNA expression-promoting agent, except for measuring the expression amount of LXRα mRNA by using oligo DNAs represented by
5'-ACACCTACATGCGTCGCAAGT-3' (SEQ ID NO: 4),
5'-TCTTGCCGCTTCAGTTTCTTC-3' (SEQ ID NO: 5) and
5'-TGTGTCCTGTCAGAAGAACAGATCCGCC-3' (SEQ ID NO: 6)
(designed using the Primer Express Program provided by Applied Biosystems) are used as a sense strand primer, an antisense strand primer and a TaqMan probe, respectively.

The activity of the ABCG1 mRNA expression-promoting agent of the present invention can be confirmed, for example, according to the following method.

The activity of the ABCG1 mRNA expression-promoting agent of the present invention can be confirmed by a similar method to that for the above-mentioned ABCA1 mRNA expression-promoting agent, except for measuring the expression amount of ABCG1 mRNA by using oligo DNAs represented by
5'-TGGAAAATGCCAAGCTGTACCT-3' (SEQ ID NO: 7),
5'-CGGATTTTGTACCTGAGGAC-3' (SEQ ID NO: 8) and
5'-TTCATCTCCCTCCGCCTCATTGCCTA-3' (SEQ ID NO: 9) (designed using the Primer Express Program provided by Applied Biosystems) are used as a sense strand primer, an antisense strand primer and a TaqMan probe, respectively.

The activity of the cholesterol efflux-promoting agent of the present invention can be confirmed, for example, according to the following method.

Human-derived cells are suspended into a PMA (phorbol 12-myristate 13-acetate)-containing RPMI1640 medium (containing serum), dispensed in plates and cultured until differentiation.

The medium is removed, and [³H]cholesteryl linolate-β-VLDL-OA-BSA-containing RPMI1640 medium, to which a test compound has been added, is added to the plate and cultured.

The plate is washed with a buffer solution, and then a RPMI1640 medium containing a test compound and apoAl is added thereto and cultured.

Thus-obtained culture solution is centrifuged. The radioactivity of the supernatant is measured using a liquid scintillation counter to confirm the cholesterol efflux-promoting activity of the test compound.

### BEST MODE FOR CARRYING OUT THE INVENTION

Hereinafter the present invention is more specifically explained with referring to Examples and Experimental Examples.

The SEQ ID NOs in the Sequence Listing of the present description represent the following sequences.
[SEQ ID NO: 1] shows a sense strand primer, which is used to amplify cDNA encoding human ABCA1.
[SEQ ID NO: 2] shows an antisense strand primer, which is used to amplify cDNA encoding human ABCA1.
[SEQ ID NO: 3] shows a TaqMan probe, which is used to quantify cDNA encoding human ABCA1.
[SEQ ID NO: 4] shows a sense strand primer, which is used to amplify cDNA encoding human LXRα.
[SEQ ID NO: 5] shows an antisense strand primer, which is used to amplify cDNA encoding human LXRα.
[SEQ ID NO: 6] shows a TaqMan probe, which is used to quantify cDNA encoding the amplified human LXRα.
[SEQ ID NO: 7] shows a sense strand primer, which is used to amplify cDNA encoding human ABCG1.
[SEQ ID NO: 8] shows an antisense strand primer, which is used to amplify cDNA encoding human ABCG1.
[SEQ ID NO: 9] shows a TaqMan probe, which is used to quantify cDNA encoding the amplified human ABCG1.
[SEQ ID NO: 10] shows a sense strand primer, which is used to amplify cDNA encoding mouse LXRα.
[SEQ ID NO: 11] shows an antisense strand primer, which is used to amplify cDNA encoding mouse LXRα.
[SEQ ID NO: 12] shows a TaqMan probe, which is used to quantify cDNA encoding the amplified mouse LXRα.
[SEQ ID NO: 13] shows a sense strand primer, which is used to amplify cDNA encoding mouse ABCG1.
[SEQ ID NO: 14] shows an antisense strand primer, which is used to amplify cDNA encoding mouse ABCG1.
[SEQ ID NO: 15] shows a TaqMan probe, which is used to quantify cDNA encoding the amplified mouse ABCG1.
[SEQ ID NO: 16] shows a sense strand primer, which is used to amplify cDNA encoding mouse acidic ribosomal protein (36B4).
[SEQ ID NO: 17] shows an antisense strand primer, which is used to amplify cDNA encoding mouse acidic ribosomal protein (36B4).
[SEQ ID NO: 18] shows a TaqMan probe, which is used to quantify cDNA encoding the amplified mouse acidic ribosomal protein (36B4).
[SEQ ID NO: 19] shows a sense strand primer, which is used to amplify cDNA encoding human ACAT-1.
[SEQ ID NO: 20] shows an antisense strand primer, which is used to amplify cDNA encoding human ACAT-1.
[SEQ ID NO: 21] shows a TaqMan probe, which is used to quantify cDNA encoding the amplified human ACAT-1.
[SEQ ID NO: 22] shows a sense strand primer, which is used to amplify cDNA encoding human CEH.
[SEQ ID NO: 23] shows an antisense strand primer, which is used to amplify cDNA encoding human CEH.
[SEQ ID NO: 24] shows a TaqMan probe, which is used to quantify cDNA encoding the amplified human CEH.

### EXAMPLES

### Example 1 (Production of tablets)

Pioglitazone hydrochloride (2479.5 g: 2250 g as pioglitazone), lactose (13930.5 g) and carboxymethylcellulose calcium (carmelose calcium) (540 g) were fed into a fluidized-bed granulation and drying machine (manufactured by Powerex Corporation), mixed with preheating, and sprayed with an aqueous solution (7500 g) in which hydroxypropylcellulose (450 g) had been dissolved, to give granulated powder. The obtained granulated powder (16820 g) was passed through a cutter mill (manufactured by Showa Kagaku Kikai Kosakusho) to give sized powder. The obtained sized powder (16530 g), carmelose calcium (513 g) and magnesium stearate (57 g) were formed into mixed powder using a tumbler mixer (manufactured by Showa Kagaku Kikai Kosakusho), and then the mixed powder (16800 g) was compressed into tablets by a tableting machine (manufactured by Kikusui Seisakusho) to give 140,000 tablets containing 15 mg of pioglitazone per a tablet and having the following composition.

### Composition of a tablet (unit: mg):

| | |
|---|---|
| 1) pioglitazone hydrochloride | 16.53 |
| 2) lactose | 92.87 |
| 3) carmelose calcium | 7.2 |
| 4) hydroxypropylcellulose | 3.0 |
| 5) magnesium stearate | 0.4 |
| Total | 120.0 |

### Example 2 (Production of tablets)

According to the similar method to Example 1, 140,000 tablets containing 30 mg of pioglitazone per a tablet and having the following composition were obtained.

### Composition of a tablet (unit: mg):

| | |
|---|---|
| 1) pioglitazone hydrochloride | 33.06 |
| 2) lactose | 76.34 |
| 3) carmelose calcium | 7.2 |
| 4) hydroxypropylcellulose | 3.0 |
| 5) magnesium stearate | 0.4 |
| Total | 120.0 |

### Example 3 (Production of tablets)

According to the similar method to Example 2, 140,000 tablets containing 45 mg of pioglitazone per a tablet and having the following composition were obtained.

### Composition of a tablet (unit: mg):

| | |
|---|---|
| 1) pioglitazone hydrochloride | 49.59 |
| 2) lactose | 114.51 |
| 3) carmelose calcium | 10.8 |
| 4) hydroxypropylcellulose | 4.5 |
| 5) magnesium stearate | 0.6 |
| Total | 180.0 |

### Example 4 (Production of film tablets)

### [Production of coating agents]

To purified water (4859.1 g) were dissolved hydroxypropylmethyl cellulose 2910(TC-5) (403.4 g) and polyethyleneglycol 6000 (81.0 g). Into the obtained solution were dispersed titanium oxide (54.0 g) and yellow iron sesquioxide (1.620 g) to give a coating agent.

The following embodiments related to [Production of bare tablets] and to [Production of film coated tablets] do not form part of the invention but represent background art useful for understanding the invention.

### [Production of bare tablets]

Compound (7) (576.0 g), lactose (2513 g), corn starch (356.4 g) and croscarmellose sodium (217.8 g) were fed into a fluidized-bed granulation and drying machine (manufactured by Powerex Corporation), mixed with preheating, and sprayed with an aqueous solution (1963 g) in which hydroxypropylcellulose (138.6 g) had been dissolved, to give granulated powder. The obtained granulated powder (3590 g) was passed through a power mill (manufactured by Showa Kagaku Kikai Kosakusho) to give sized powder. The obtained sized powder (3432 g), corn starch (125.1 g) and magnesium stearate (17.88 g) were mixed in a tumbler mixer (manufactured by Showa Kagaku Kikai Kosakusho), and then the obtained mixed powder (3410 g) was compressed into tablets by a tableting machine (manufactured by Kikusui Seisakusho) to give bare tablets.

### [Production of film coating tablets]

The obtained bare tablets (27000 tablets) was sprayed with the above-mentioned coating agent in a film coating machine (manufactured by Powerex Corporation) to give 27,000 tablets of film coating tablets containing 16.0 mg of the compound (7) per a tablet and having the following formulation.

### Formulation of a tablet (composition per a tablet):

(Bare tablet)

| | |
|---|---|
| 1) compound (7) | 16.0 mg |
| 2) lactose | 69.8 mg |
| 3) corn starch | 13.75 mg |
| 4) croscarmellose sodium | 6.05 mg |
| 5) hydroxypropylcellulose | 3.85 mg |
| 6) magnesium stearate | 0.55 mg |
| Total | 110.0 mg |

(Film component)

| | |
|---|---|
| 7) hydroxypropylmethyl cellulose 2910 | 2.988 mg |
| 8) polyethyleneglycol 6000 | 0.6 mg |
| 9) titanium oxide | 0.4 mg |
| 10) yellow iron sesquioxide | 0.012 mg |
| Total | 114.0 mg |

### Example 5 (Production of film tablets)

### [Production of bare tablets]

According to the similar method to Example 4 except that the amount to be used of the compound (7) and lactose were 144.0 g and 2945 g respectively, 27,000 tablets of film coating tablets containing 4.0 mg of the compound (7) per a tablet and having the following formulation were obtained.

### Formulation of a tablet (Composition per a tablet):

(Bare tablet)

| | |
|---|---|
| 1) compound (7) | 4.0 mg |
| 2) lactose | 81.8 mg |
| 3) corn starch | 13.75 mg |
| 4) croscarmellose sodium | 6.05 mg |
| 5) hydroxypropylcellulose | 3.85 mg |
| 6) magnesium stearate | 0.55 mg |
| Total | 110.0 mg |

(Film component)

| | |
|---|---|
| 7) hydroxypropylmethyl cellulose 2910 | 2.988 mg |
| 8) polyethyleneglycol 6000 | 0.6 mg |
| 9) titanium oxide | 0.4 mg |
| 10) yellow iron sesquioxide | 0.012 mg |
| Total | 114.0 mg |

### Example 6 (Production of film tablets)

### [production of bare tablets]

According to the similar method to Example 4 except that the amount to be used of the compound (7) and lactose were 36.0 g and 3053 g respectively, 27,000 tablets of film coating tablets containing 1.0 mg of the compound (7) per a tablet and having the following formulation were obtained.

### Formulation of a tablet (Composition per a tablet):

(Bare tablet)

| | |
|---|---|
| 1) compound (7) | 1.0 mg |
| 2) lactose . | 84.8 mg |
| 3) corn starch | 13.75 mg |
| 4) croscarmellose sodium | 6.05 mg |
| 5) hydroxypropylcellulose | 3.85 mg |
| 6) magnesium stearate | 0.55 mg |
| Total | 110.0 mg |

(Film component)

| | |
|---|---|
| 7) hydroxypropylmethyl cellulose 2910 | 2.988 mg |
| 8) polyethyleneglycol 6000 | 0.6 mg |
| 9) titanium oxide | 0.4 mg |
| 10) yellow iron sesquioxide | 0.012 mg |
| Total | 114.0 mg |

Experimental Example 1 (Expression modulatory activities for LXRα mRNA, ABCA1 mRNA and ABCG1 mRNA in human monocyte-derived cells)

To a PMA (phorbol 12-myristate 13-acetate, 400ng/mL)-FBS (fetal bovine serum, 10%)-containing RPMI medium was suspended human monocyte-derived THP-1 cells (ATCC) (7x10⁵ cell/mL), and the obtained suspension was dispensed into a 24-well plate (0.5 mL/well) and cultured for 3 days. After removing the medium, a FBS (fetal bovine serum, 10%)-containing RPMI1640 medium into which a test compound (1 µM) had been added was added to the 24-well plate and the plate was cultured for 24 hours. Whole RNA was extracted from the cultured cells using RNeasy Mini Kit (QIAGEN), and then the expression amounts of LXRα mRNA, ABCA1 mRNA, ABCG1 mRNA and Human acidic ribosomal protein (huPO; 36B4) mRNA as an internal standard were measured by real-time quantitative RT-PCR according to TaqMan PCR method using a TaqMan EZ RT-PCR kit (Applied Biosystems).

In the above-mentioned method, for the measurement of the expression amount of LXRα mRNA, the above-mentioned oligo DNAs represented by SEQ ID NO: 4, SEQ ID NO: 5 and SEQ ID NO: 6 (designed using the Primer Express Program provided by Applied Biosystems) were used as a sense strand primer, an antisense strand primer and a TaqMan probe, respectively.

Furthermore, for the measurement of the expression amount of ABCA1 mRNA, the above-mentioned oligo DNAs represented by SEQ ID NO: 1, SEQ ID NO: 2 and SEQ ID NO: 3 (designed using the Primer Express Program provided by Applied Biosystems) were used as a sense strand primer, an antisense strand primer and a TaqMan probe, respectively.

Moreover, for the measurement of the expression amount of ABCG1 mRNA, the above-mentioned oligo DNAs represented by SEQ ID NO: 7, SEQ ID NO: 8 and SEQ ID NO: 9 (designed using the Primer Express Program provided by Applied Biosystems) were used as a sense strand primer, an antisense strand primer and a TaqMan probe, respectively.

For the TaqMan probes used for the above-mentioned various measurements, 6-carboxyfluorescein (6FAM) as a reporter dye was added to the 5'-terminus, and 6-carboxy-N,N,N',N'-tetramethylrhodamine (TAMRA) as a quencher dye was added to the 3'-terminus.

The expression amount of Human acidic ribosomal protein (huPO; 36B4) mRNA was measured using Pre-Developed TaqMan Assay Reagents Endogenous Control (Human Acidic ribosomal protein (huPO)) (Applied Biosystems), and VIC was used as a reporter dye and TAMRA was used as a quencher dye.

Real-time quantitative RT-PCR is carried out by 40 cycles, wherein one cycle comprises reaction of 50°C for 2 minutes and at 60°C for 30 minutes, keeping at 95°C for 5 minutes and then at 94°C for 20 seconds, and reaction at 60°C for 1 minute, using ABI PRISM 7700 Sequence Detector (Applied Biosystems).

Based on the ratio of the expression amount of the LXRα mRNA, ABCAL mRNA and ABCG1 mRNA relative to the internal standard (expression amount of Human acidic ribosomal protein mRNA), the expression modulatory activities for the LXRα mRNA, ABCA1 mRNA and ABCG1 mRNA of the test compound were evaluated. The results are shown in Table 1.

**[Table 1]**

| Test compound | Expression amount of mRNA | | |
|---|---|---|---|
| | (Relative ratio % to internal standard) | | |
| | LXRα | ABCA1 | ABCG1 |
| Not added | 100.0±7.1 | 100.0±7.1 | 100.0±11.7 |
| Pioglitazone hydrochloride | 165.7±4.6 | 108.2±3.5 | 252.0±9.2 |

| | | | |
|---|---|---|---|
| Mean±SEM (n=3) | | | |

As shown in Table 1, pioglitazone hydrochloride increased the expression amounts of LXRα mRNA, ABCA1 mRNA and ABCG1 mRNA.

### Experimental Example 2 (Expression modulatory activities for LXRα mRNA and ABCG1 mRNA in mouse macrophages)

A C57BL6/J mouse (male, 10 weeks old) was intraperitoneally administered with 1 mL of a 5% thioglycolic acid medium (Wako Pure Chemical Industries, Ltd.). After 4 days, intraperitoneal cells were collected with phosphate-buffered saline (PBS), and macrophages were separated in a 3%BSA-containing DMEM medium as cells adhesive to a plate. After incubation of the macrophages overnight in a DMEM medium containing 10% human lipoprotein-removed serum, the medium was replaced with a DMEM medium containing a test compound (1 µM) and 10% fetal bovine serum and cultured for 24 hours.

After the medium was removed, whole RNA was extracted from the obtained macrophage using RNeasy Mini Kit (QIAGEN) and then the expression amounts of the LXRαmRNA, ABCG1mRNA and mouse acidic ribosomal protein (36B4) mRNA as an internal standard were measured by real-time quantitative RT-PCR according to TaqMan PCR method using a TaqMan EZ RT-PCR kit (Applied Biosystems).

In the above mentioned method, for the measurement of the expression amount of the LXRα mRNA, oligo DNAs represented by
5'- AGCAACAGTGTAACAGGCGCT -3' (SEQ ID NO: 10),
5'- ACGATGGCCAGCTCAGTAAAG -3' (SEQ ID NO: 11) and
5'- CTCAGACCGCCTGCGCGTCA -3' (SEQ ID NO: 12)
(designed using the Primer Express Program provided by Applied Biosystems) were used as a sense strand primer, an antisense strand primer and a TaqMan probe, respectively.

Furthermore, for the measurement of the expression amount of the ABCG1 mRNA, oligo DNAs represented by
5'- TCAGGAGGCCATGATGGTG -3' (SEQ ID NO: 13),
5'- CCCGTCTGCCTTCATCCTT -3' (SEQ ID NO: 14) and
5'- CCGCGCATCTGAAGCTGCAGG -3' (SEQ ID NO: 15)
(designed using the Primer Express Program provided by Applied Biosystems) were used as a sense strand primer, an antisense strand primer and a TaqMan probe, respectively.

For the TaqMan probes used for the above-mentioned various measurements, 6-carboxyfluorescein (6FAM) as a reporter dye was added to the 5'-terminus, and 6-carboxy-N,N,N',N'-tetramethylrhodamine (TAMRA) as a quencher dye was added to the 3'-terminus.

For the measurement of the expression amount of mouse acidic ribosomal protein (36B4) mRNA, oligo DNAs represented by
5'- AGACCTCCTTCTTCCAGGCTTT -3' (SEQ ID NO: 16),
5'- GCTGCACATCACTCAGAATTTCA -3' (SEQ ID NO: 17) and
5'- TCACCACGAAAATCTCCAGAGGCACC -3' (SEQ ID NO: 18)
(designed using the Primer Express Program provided by Applied Biosystems) were used as a sense strand primer, an antisense strand primer and a TaqMan probe, respectively. In the TaqMan probes, VIC as a reporter dye was added to the 5'-terminus and TAMRA as a quencher dye was added to the 3'-terminus.

Real-time quantitative PCR was carried out according to the similar manner to Experimental Example 1.

Based on the ratio of the expression amount of the LXRαmRNA and ABCG1mRNA relative to the internal standard (expression amount of mouse acidic ribosomal protein mRNA), the expression modulatory activities for the LXRα mRNA and ABCG1 mRNA of the test compound were evaluated. The results are shown in Table 2.

**[Table 2]**

| Test compound | Expression amount of mRNA | |
|---|---|---|
| | (Relative ratio % to internal standard) | |
| | LXRα | ABCG1 |
| Not added | 100.0±13.6 | 100.0±4.2 |
| Pioglitazone hydrochloride | 145.3±17.9 | 195.2±23.1 |

| | | |
|---|---|---|
| Mean±SEM (n=3) | | |

As shown in Table 2, pioglitazone hydrochloride increased the expression amounts of LXRα mRNA and ABCG1 mRNA.

### Experimental Example 3 (Foaming-suppressant activity in human monocyte-derived cells)

In a RPMI1640 medium containing a test compound (1 µM), PMA (phorbol 12-myristate 13-acetate, 400 ng/ml) and 10% fetal bovine serum was suspended human monocyte-derived THP-1 cells (purchased from Dainippon Pharmaceutical Co., Ltd.). The obtained suspension was dispensed into a 12-well plate (7.2x10⁵ cells) and was cultured for 3 days.
The obtained cell was further cultured in a RPMI1640 medium containing the test compound and 0.2 mM oleic acid-0.2% bovine serum albumin complex and rabbit β-VLDL (200 µg cholesterol/mL) for 2 days, and then the total lipids in the cells were extracted with a mixed solution of n-hexane/isopropanol (3:2). The amounts of the total cholesterol (TC) and free cholesterol (FC) in the extract were measured with a enzyme method [using Cholesterol E-Test Wako (trade name, Wako Pure Chemical Industries, Ltd.)], and the amount of cholesteryl ester (CE) was calculated by the difference between the two cholesterol amounts. The intracellular lipid content was then evaluated by dividing the amounts of these lipids by the amount of cell protein (measured using BCA assay kit (Pierce)). The results are shown in Table 3. In the Table, TC represents total cholesterol, FC represents free cholesterol and CE represents cholesteryl ester.

**[Table 3]**

| Test compound | Intracellular lipid content | | |
|---|---|---|---|
| | (µg/mg cell protein) | | |
| | TC | FC | CE |
| Not added | 114.8±1.8 | 80.8±1.2 | 34.0±2.3 |
| Pioglitazone hydrochloride | 100.3±0.3 | 72.7±0.4 | 27.6±0.5 |

| | | | |
|---|---|---|---|
| Mean±SEM (n=3) | | | |

As shown in Table 3, pioglitazone hydrochloride decreased the amounts of total cholesterol, free cholesterol and cholesteryl ester in cells. Based on the decrease in the amount of cholesteryl ester, it was revealed that pioglitazone hydrochloride have foaming-suppressant activity.

### Experimental Example 4 (Expression modulatory activities for ACAT-1 mRNA and CEH mRNA in human monocyte-derived cells)

In a RPMI medium containing a test compound (1 µM), PMA (phorbol 12-myristate 13-acetate, 400ng/mL)-FBS (fetal bovine serum, 10%) was suspended human monocyte-derived THP-1 cells (purchased from-Dainippon Pharmaceutical Co., Ltd.) (7.2x10⁵ cell/mL). The obtained suspension was dispensed into a 24 well plate (0.5 mL/well) and cultured for 2 days (3.6x10⁵ cells). After the medium was removed, a RPMI medium containing the test compound (1 µM), PMA(400ng/mL)-FBS(10%) was added to the plate and it was cultured further for 1 day.

After the medium was removed, whole RNA was extracted from the obtained cells using RN easy Mini Kit (QIAGEN), and the expression amounts of ACAT-1 mRNA, CEH mRNA and human acidic ribosomal protein (huPO; 36B4) mRNA as an internal standard were measured by real-time quantitative RT-PCR according to TaqMan PCR method using a TaqMan EZ RT-PCR kit (Applied Biosystems).

In the above mentioned method, for the measurement of the expression amount of the ACAT-1mRNA, oligo DNAs represented by
5'- GCAGCTGCTCTGGTTCTCATG -3' (SEQ ID NO: 19),
5'- TTCTTGCCAGTGGTGTAACATTG -3' (SEQ ID NO: 20) and
5'- CGGCAGATGCAGCGAAGAGGC -3' (SEQ ID NO: 21) (designed using the Primer Express Program provided by Applied Biosystems) were used as a sense strand primer, an antisense strand primer and a TaqMan probe, respectively.

Furthermore, for the measurement of the expression amount of the CEH mRNA, oligo DNAs represented by
5'- AGCCCTTGGCTGAGCAAA -3' (SEQ ID NO: 22),
5'- GTCGCAGGCAGTGAACCAT -3' (SEQ ID NO: 23) and
5'- TGCTATCACTGCTGGGTGCAAAACCA -3' (SEQ ID NO: 24)
(designed using the Primer Express Program provided by Applied Biosystems) were used as a sense strand primer, an antisense strand primer and a TaqMan probe, respectively.

For the TaqMan probes used for the above-mentioned various measurements, 6-carboxyfluorescein (6FAM) as a reporter dye was added to the 5'-terminus and 6-carboxy-N,N,N',N'-tetramethylrhodamine (TAMRA) as a quencher dye was added to the 3'-terminus.

The expression amount of human acidic ribosomal protein (huPO; 36B4) mRNA was measured according to the method similar to Experimental Example 1. Real-time quantitative RT-PCR was carried out similarly to

### Experimental Example 1.

Based on the ratio of the expression amounts of the ACAT-1 mRNA and CEH mRNA relative to the internal standard (expression amount of human acidic ribosomal protein mRNA), the expression modulatory activities for the ACAT-1 mRNA and CEH mRNA of the test compound were evaluated. The results are shown in Table 4.

**[Table 4]**

| Test compound | Expression amount of mRNA | |
|---|---|---|
| | (Relative ratio % to internal standard) | |
| | ACAT-1 | CEH |
| Not added | 100.0±8.6 | 100.0±13.0 |
| Pioglitazone hydrochloride | 65.8±1.9 | 148.6±9.2 |

| | | |
|---|---|---|
| Mean±SEM(n=3) | | |

As shown in Table 4, pioglitazone hydrochloride and decreased the expression amount of ACAT-1 mRNA and increased the expression amount of CEH mRNA.

### INDUSTRIAL APPLICABILITY

The ABCA1 mRNA expression promoting agent, LXRα mRNA expression promoting agent, ABCG1 mRNA expression-promoting agent, cholesterol efflux-promoting agent, cholesteryl ester accumulation-inhibiting agent, ACAT-1 mRNA expression-inhibiting agent and CEH mRNA expression-promoting agent of the present invention are excellent in the ability to control cholesterol distribution in the body and have low toxicity.

## Claims

1. Pioglitazone or a salt thereof for use in treatment of arteriosclerosis obliterans.

2. Use of pioglitazone or a salt thereof for the manufacture of a medicament for treating arteriosclerosis obliterans.

3. Agent comprising pioglitazone or a salt thereof for use in treatment of arteriosclerosis obliterans.

## Patentansprüche

1. Pioglitazon oder ein Salz davon zur Verwendung bei der Behandlung von Arteriosklerose obliterans.

2. Verwendung von Pioglitazon oder einem Salz davon für die Herstellung eines Medikaments zur Behandlung von Arteriosklerose obliterans.

3. Pioglitazon oder ein Salz davon umfassendes Mittel zur Verwendung bei der Behandlung von Arteriosklerose obliterans.

## Revendications

1. Pioglitazone ou l'un de ses sels, pour son utilisation dans le traitement de l'artériosclérose oblitérante.

2. Utilisation de la pioglitazone ou de l'un de ses sels, pour la fabrication d'un médicament pour le traitement de l'artériosclérose oblitérante.

3. Agent comprenant de la pioglitazone ou de l'un de ses sels, pour son utilisation dans le traitement de l'artériosclérose oblitérante.
